# EUROPEAN PATENT SPECIFICATION

(11) **EP 3 453 310 B1**
(45) Date of publication and mention of the grant of the patent: **20.04.2022**
(21) Application number: 18192659.3
(22) Date of filing: 05.09.2018
(51) Int. Cl.: A61B 1/00, A61B 1/005, A61B 1/015, A61B 1/12

(54) **ENT GUIDEWIRE WITH CAMERA ON TIP**
ENT-FÜHRUNGSDRAHT MIT KAMERA AUF SPITZE
FIL-GUIDE OTO-RHINOLARYNGOLOGIQUE AVEC CAMÉRA SUR POINTE

(30) Priority: 06.09.2017 US 201762554922 P; 12.08.2018 US 201816101503
(43) Date of publication of application: 13.03.2019
(73) Proprietor: Biosense Webster (Israel) Ltd., Yokneam 2066717 (IL)
(72) Inventor: GOVARI, Assaf, Yokneam, Israel 2066717 (IL); ALGAWI, Yehuda, Yokneam, Israel 2066717 (IL); SITNITSKY, Ilya, Yokneam, Israel 2066717 (IL)
(74) Representative: Carpmaels & Ransford LLP

(56) References cited:
- EP-A1- 2 859 835
- WO-A1-2017/083648
- US-A1- 2013 331 730

## Description

### FIELD OF THE INVENTION

This invention relates generally to surgical tools, and specifically to a surgical tool used for ENT (ear, nose, and throat) procedures.

### BACKGROUND OF THE INVENTION

Prior to, or during the performance of, an ENT procedure such as a balloon sinuplasty, it is advantageous for the physician performing the procedure to be presented, if possible, with images of the region being operated on. This is typically difficult in the case of sinuplasty, because of the narrow and tortuous pathways of the sinuses, and because they are relatively close to sensitive organs such as the optic nerves.

US 2013/331730 A1 relates to an anatomical probe system that comprises an elongated flexible body and an operational component extending within a channel of the flexible body. The system also comprises a support member at a distal end of the elongated flexible body. The support member includes a fluid director adapted to direct a fluid from the channel toward the operational component.

WO 2017/083648 A1 ,which discloses an apparatus according to the preamble of independent claim 1 and a method according to the preamble of independent claim 6, relates to a semi-rigid endoscope that may include a shaft that is elongate along a longitudinal direction. The shaft including a shaft body that defines: proximal rigid portion and a flexible tip that is distal to the proximal rigid portion. The flexible tip moves along a lateral direction that is perpendicular to the longitudinal direction. The flexible tip moves along a transverse direction that is perpendicular to both the longitudinal and lateral directions. The endoscope may also include at least one conduit that extends longitudinally along an internal portion of the shaft body.

EP 2859835 A1 relates to an insert section of an electronic endoscope that has an air and water nozzle, a forceps outlet, an observation window, and a fluid guide slope at its distal portion. The fluid guide slope situated in an ejection area of fluid ejected from the air and water nozzle connects a flat surface to the forceps outlet in an inclined manner relative to the flat surface of the distal portion.

### SUMMARY OF THE INVENTION

The present invention provides apparatus, including:
a guidewire having a proximal end and a distal end which is deflectable;
at least one wire within the guidewire, traversing the guidewire from the proximal end to the distal end, wherein the at least one wire is fixed to the distal end;
one or more irrigation tubes within the guidewire and traversing the guidewire from the proximal end to the distal end; and
an imaging assembly enclosure, fixedly attached to the distal end, having:
   a camera, having a distal face, fixedly mounted within the enclosure;
   respective deflectors for each of the one or more irrigation tubes, fixedly attached to and positioned at a distal end of the enclosure so as to deflect fluid, ejected from the one or more irrigation tubes, to pass over the camera distal face so as to clean the face,
   so that applying tension at the proximal end of the guidewire on the at least one wire deflects the distal end of the guidewire, the imaging assembly enclosure attached thereto, and the camera in the enclosure; and wherein the one or more irrigation tubes comprises at least two irrigation tubes on opposite sides of the camera, and wherein the at least two irrigation tubes are configured to simultaneously eject and withdraw fluid from the camera distal face.

In a disclosed embodiment the distal face of the camera is orthogonal to a surface of the enclosure, and each of the respective deflectors has a first straight section orthogonal to the distal face of the camera and connected to the distal end of the enclosure, a second straight section parallel to the distal face of the camera, and a curved section connecting the first and second straight sections. Each of the one or more irrigation tubes may have a respective distal end parallel to the first straight section of the each of the respective deflectors.

In a further disclosed embodiment the application of suction to a given tube of the two or more irrigation tubes causes the respective deflector of the given tube to remove fluid from the camera distal face, and to direct the fluid into the given tube.

In a yet further disclosed embodiment the apparatus includes a magnetic sensor, mounted in the imaging assembly enclosure, that is configured to provide an indication of a location and an orientation of the imaging assembly enclosure in response to a magnetic field traversing the sensor.

There is further provided, according to an embodiment of the present invention, a method, including:
providing a guidewire having a proximal end and a distal end which is deflectable;
positioning at least one wire within the guidewire, to traverse the guidewire from the proximal end to the distal end, wherein the at least one wire is fixed to the distal end;
positioning one or more irrigation tubes within the guidewire to traverse the guidewire from the proximal end to the distal end; and
fixedly attaching an imaging assembly enclosure to the distal end, the assembly having:
   a camera, having a distal face, fixedly mounted within the enclosure;
   respective deflectors for each of the one or more irrigation tubes, fixedly attached to and positioned at a distal end of the enclosure so as to deflect fluid, ejected from the one or more irrigation tubes, to pass over the camera distal face so as to clean the face,
   so that applying tension at the proximal end of the guidewire on the at least one wire deflects the distal end of the guidewire, the imaging assembly enclosure attached thereto, and the camera in the 2. enclosure; and wherein the one or more irrigation tubes comprises at least two irrigation tubes on opposite sides of the camera, and wherein the at least two irrigation tubes are configured to simultaneously eject and withdraw fluid from the camera distal face.

The present disclosure will be more fully understood from the following detailed description of the embodiments thereof, taken together with the drawings, in which:

### BRIEF DESCRIPTION OF THE DRAWINGS

Fig. 1 is a schematic illustration of an ENT (ear, nose, and throat) system, according to an embodiment of the present invention;
Fig. 2 is a schematic diagram of a tool, a guidewire, and a balloon insertion mechanism, according to an embodiment of the present invention;
Fig. 3 is a schematic diagram of portions of the tool and of the guidewire, according to an embodiment of the present invention;
Figs. 4 and 5 are schematic detail figures of a knob and its internal construction, according to an embodiment of the present invention; and
Figs. 6, 7A, 7B, 8, and 9 are schematic figures illustrating the guidewire and elements associated with the guidewire, according to an embodiment of the present invention

### DETAILED DESCRIPTION OF EMBODIMENTS

### Overview

An embodiment of the present invention provides a physician performing an ENT (ear nose and throat) procedure with a tool that can be used to perform a balloon sinuplasty procedure, using a guidewire that is inserted into the patient for the purpose of the physician viewing internal organs, such as the sinuses, of the patient. The procedure involves using a balloon insertion mechanism which is slid over the guidewire to a desired location in the patient.

The physician may insert the distal end of the tool into the patient's nostril, and manipulate the distal end to enable access to a desired sinus region. The guidewire, which at its distal tip comprises a camera, illuminators for the camera, and a location sensor in an imaging assembly, may then be threaded through the tool. The guidewire also comprises one or more wires attached to the distal tip, and by applying tension to the wires the physician may deflect the distal tip, and thus manipulate the imaging assembly to observe the patient sinuses. The physician typically uses the guidewire before, during, and after using the balloon insertion mechanism to perform a balloon sinuplasty procedure on the patient.

Irrigation tubes traverse the guidewire, and the distal ends of the tubes terminate approximately in line with a distal face of the camera. The irrigation tubes permit irrigation fluid to be injected into the tubes, and the camera assembly comprises deflectors opposite the respective tube distal ends. The deflectors are configured to direct irrigation fluid, ejecting from the tube distal ends, so it passes across the camera distal face. The irrigation tubes may also be configured to withdraw fluid, also directed by the deflectors, from the camera distal face.

The irrigation fluid traversing the camera distal face cleans the camera face of any mucus or blood, or other nontransparent material. The presence of fluid such as mucus or blood renders the camera substantially blind, while using the deflected irrigation fluid returns the camera to an operative state. The cleaning effect may be enhanced if, while some of the tubes are configured to provide deflected irrigation fluid across the camera face, other tubes are configured to withdraw fluid from the camera face.

An embodiment of the present invention thus provides a guidewire which has a proximal end, and a distal end which is deflectable. At least one wire, typically two or more wires, traverses the guidewire from the proximal end to the distal end, and the at least one wire is fixed to the distal end. Within the guidewire there are also one or more irrigation tubes that traverse the guidewire from the proximal to the distal end.

An imaging assembly enclosure is fixedly attached to the distal end, and the assembly comprises a camera, having a distal face, that is fixedly mounted within the enclosure. The enclosure also comprises deflectors, corresponding in number to the irrigation tubes, that are fixedly attached to and positioned at a distal end of the enclosure so as to deflect fluid, ejected from the one or more irrigation tubes, to pass over the camera distal face so as to clean the face.

Applying tension at the proximal end of the guidewire on the at least one wire deflects the distal end of the guidewire. Because the imaging assembly enclosure is fixedly attached to the distal end, the assembly and its camera also deflect, so that a physician applying the tension can manipulate the camera to observe the patient sinuses.

### Detailed Description

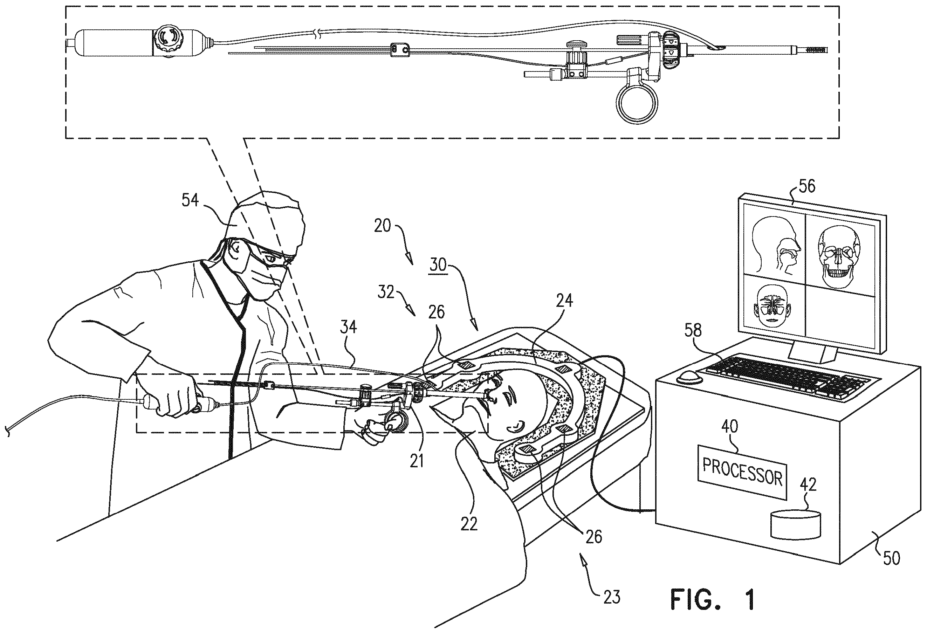

Reference is now made to Fig. 1, which is a schematic illustration of an ENT (ear, nose, and throat) system 20, according to an embodiment of the present invention. In the following description an ENT tool 21 in system 20 is assumed to be configured to typically perform two functions:
- to insert a guidewire 34 with a camera on its tip into a patient 22; and/or
- to perform a balloon sinuplasty procedure, or another procedure, typically a sinus procedure, on the patient.

When a balloon sinuplasty procedure is performed, tool 21 accommodates a balloon insertion mechanism, described below. The balloon insertion mechanism is typically slid over guidewire 34, once the latter is in place. It will be understood that ENT tool 21 accommodates the insertion of guidewire 34 and the insertion of the balloon insertion mechanism simultaneously. In some embodiments, only the guidewire may be inserted using the tool.

Tool 21 comprises a magnetic sensor 32 at its distal tip (as described in more detail below), and in some embodiments may comprise other magnetic sensors. The sensors are typically single axis coils or triple axis coils, that are tracked during the procedure by a magnetic tracking system 23. For the tracking to be effective, in system 20 frames of reference of a CT (computerized tomography) image of patient 22 and of magnetic tracking system 23, are registered. While the CT image may typically comprise a magnetic resonance imaging (MRI) image or a fluoroscopic image, in the description herein the image is assumed to comprise, by way of example, a fluoroscopic CT image.

Prior to and during insertion of the guidewire and/or performance of a procedure referred to above, a magnetic radiator assembly 24, comprised in the magnetic tracking system, is positioned beneath the patient's head. Assembly 24 comprises magnetic field radiators 26 which are fixed in position and which transmit alternating magnetic fields into a region 30 wherein the head of patient 22 is located. Potentials generated by a magnetic sensor such as sensor 32 in region 30, in response to the magnetic fields, enable the position and the orientation of the sensor to be measured in the magnetic tracking system's frame of reference.

By way of example, radiators 26 of assembly 24 are arranged in an approximately horseshoe shape around the head of patient 22. However, alternate configurations for the radiators of assembly 24 will be apparent to those having ordinary skill in the art, and all such configurations are assumed to be comprised within the scope of the present invention.

Prior to a procedure or insertion of the guidewire, the registration of the frames of reference of the magnetic tracking system with the CT image may be performed by positioning a magnetic sensor at known positions, such as the tip of the patient's nose, of the image. However, any other convenient system for registration of the frames of reference may be used.

Elements of system 20, including radiators 26 and sensor 32, are under overall control of a system processor 40. Processor 40 may be mounted in a console 50, which comprises operating controls 58 that typically include a keypad and/or a pointing device such as a mouse or trackball. Console 50 connects to the radiators and to sensor 32, and to other elements of guidewire 34, via one or more cables and/or wirelessly. A physician 54 uses operating controls 58 to interact with the processor while performing the insertion of the guidewire and/or the procedure using system 20. While performing the insertion and/or the procedure, the processor may present relevant results, typically showing locations of the distal tip of the guidewire, images from the camera on the guidewire distal tip, and/or sections of the balloon insertion mechanism, on a screen 56.

Processor 40 uses software stored in a memory 42 to operate system 20. The software may be downloaded to processor 40 in electronic form, over a network, for example, or it may, alternatively or additionally, be provided and/or stored on non-transitory tangible media, such as magnetic, optical, or electronic memory.

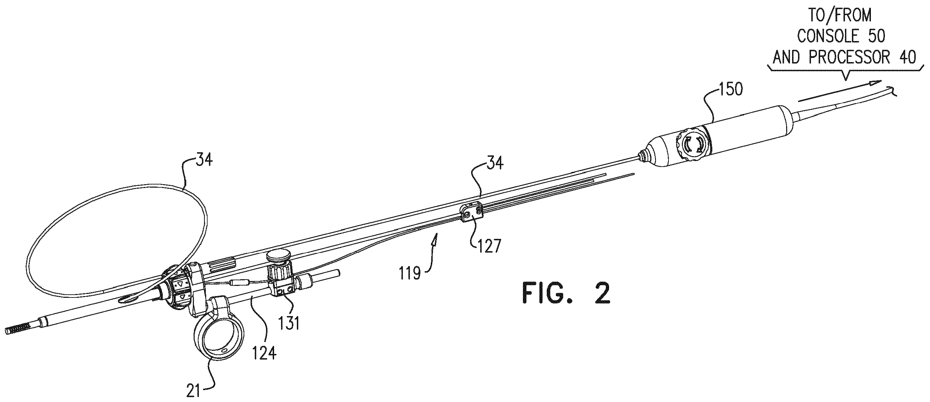

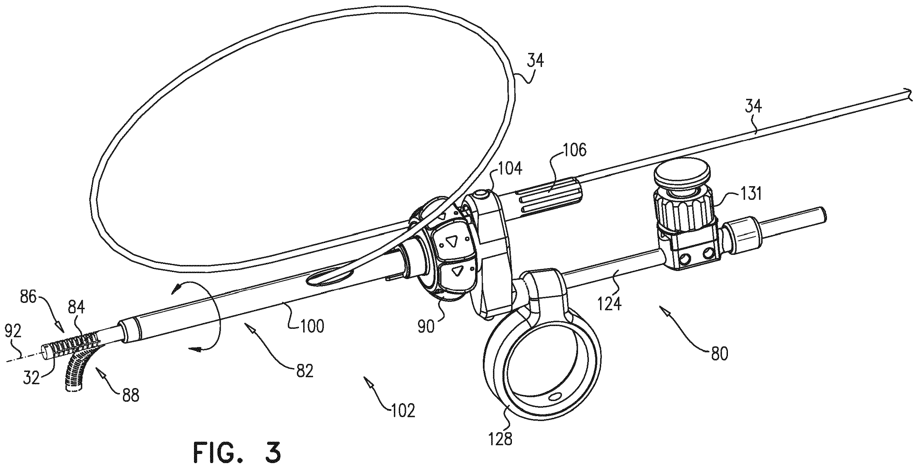

Fig. 2 is a schematic diagram of tool 21, guidewire 34, and a balloon insertion mechanism 119, and Fig. 3 is a schematic diagram of portions of the tool and of the guidewire, according to an embodiment of the present invention. For simplicity, in Fig. 2 the guidewire and the balloon insertion mechanism are both shown, in a schematic representation, after insertion into tool 21, but not exiting from the tool. However, while tool 21 accommodates the insertion of both guidewire 34 and mechanism 119 together, it will be understood that the tool may be used for insertion of either the guidewire or the mechanism.

Tool 21 comprises a proximal section 80 and a distal section 82 which are connected together, but the distal section may be disassembled and removed from the proximal section. In some embodiments the proximal and/or distal sections are designed to be disposable, typically after one procedure has been performed.

At its distal end distal section 82 comprises an articulated tubular section 84, which may be adjustably bent from a straight configuration 86 to a curved configuration 88, the latter being schematically shown in the diagram by broken lines. Magnetic sensor 32 is located at the distal tip of section 82.

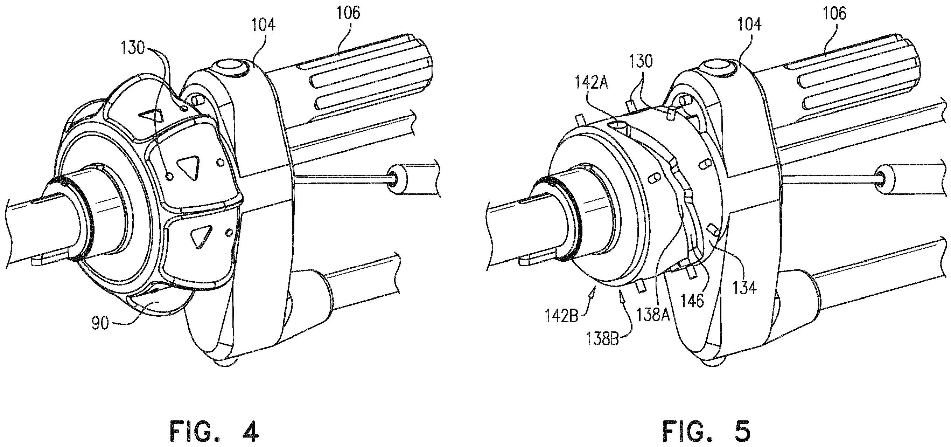

In the straight configuration tubular section 84 defines an axis of symmetry 92, which is also an axis of symmetry of a tube 100, to which section 84 is connected. The adjustment from the straight to the curved configuration, and vice versa, may be performed by clockwise and counter-clockwise rotation of a ribbed knob 90. Knob 90 is described below with respect to Figs. 4 and 5. U.S. Patent application 15/155,850, filed May 16, 2016, titled "Insertion Tube with Deflectable Tip," which is incorporated herein by reference, describes the construction and operation of a deflectable articulated section such as section 84, by rotation of an element proximal to the section, such as knob 90.

Tubular section 84 is fixedly connected at its proximal end to tube 100, and the tube and connected section 84 may be rotated about axis of symmetry 92, as indicated by the double headed arrow in the figure. The rotation of tube 100 may be implemented by rotating a ridged knob 106, the knob in turn being coupled to the tube by any convenient connection system, such as a set of gears, housed in a rotation system enclosure 104.

Tool 21 comprises a ring element 128 which adjustably connects to a suction tube 124. Suction tube 124 may be used to withdraw fluids such as mucus or blood, via enclosure 104, from tube 100 and section 34. The physician using tool 21 is able to use his/her finger or thumb to hold element 128, and thus hold the tool, so that element 128 acts as a handle for tool 21. However, any other convenient handle may be used for the tool.

Tube 100 and articulated section 84 comprise a central lumen 102 which permits the passage of guidewire 34 and balloon insertion mechanism 119 through the lumen. Proximal sections of the insertion mechanism are held in place by a manifold 127, and the sections may be locked in place, as required, by a locking mechanism 131 attached to suction tube 124.

Figs. 4 and 5 are schematic detail figures of knob 90 and its internal construction, according to an embodiment of the present invention. Knob 90, which is hidden in Fig. 5, is rigidly connected by pins 130 to a cylinder 134, and the cylinder comprises a first groove 138A and a second groove 138B at 180° to the first groove, the two grooves being configured as a double-start screw thread. A first pin 142A and a second pin 142B at 180° to the first pin are mounted on an internal cylinder 146 so that pin 142A resides within groove 138A and pin 142B resides within groove 138B. Rotation of knob 90 thus causes the knob and cylinder 134 to move in a forward or backward motion parallel to axis 92. Cylinder 134 is coupled by an internal element to wires (the internal element and the wires are not shown in the figures) connected to distal section 84, so that the motion of the cylinder parallel to axis 92 causes the distal section to bend to curved configuration 88 or straighten to straight configuration 86.

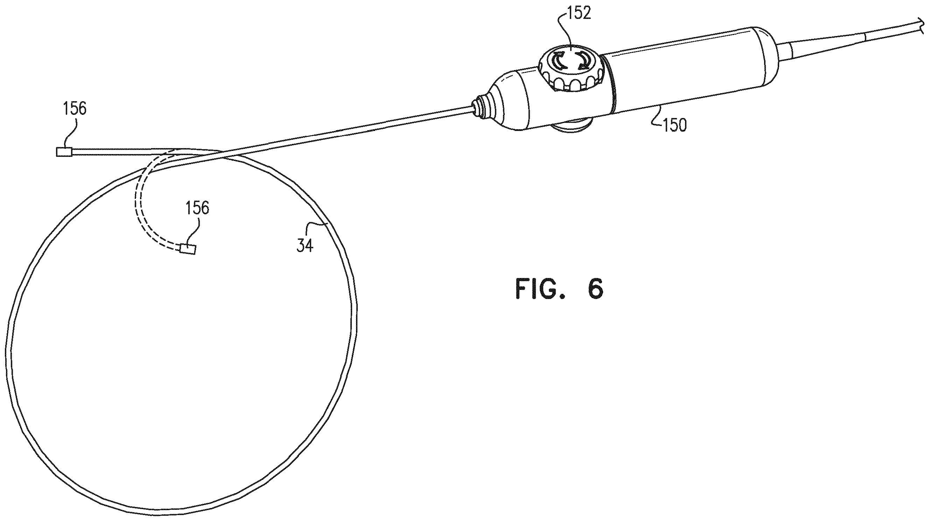

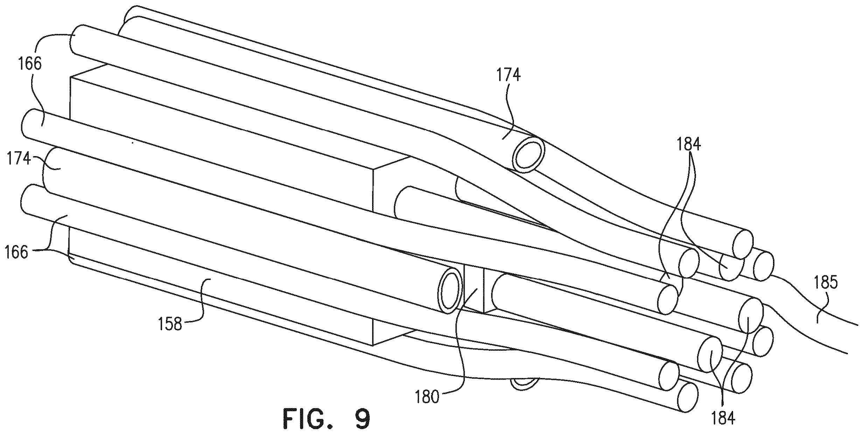

Figs. 6, 7A, 7B, 8, and 9 are schematic figures illustrating guidewire 34 and elements associated with the guidewire, according to an embodiment of the present invention. Fig. 6 illustrates a handle 150, that is connected to the guidewire at the latter's proximal end, and that is used by physician 54 in inserting the guidewire into patient 22. Handle 150 comprises a knob 152 which may be rotated so as to deflect a distal end of the guidewire between a straight state and a curved state, the latter being illustrated in the figure by broken lines. The deflection of the distal end may be implemented by at least one wire which is anchored by welding to the distal end, which traverse the guidewire, and which terminate at knob 152. Typically there are two such wires, but for simplicity only one wire 185 is shown in the figures, in Fig. 9. Handle 150 typically contains other controls for operating elements of the guidewire described herein, but for simplicity those controls are not illustrated in the figure.

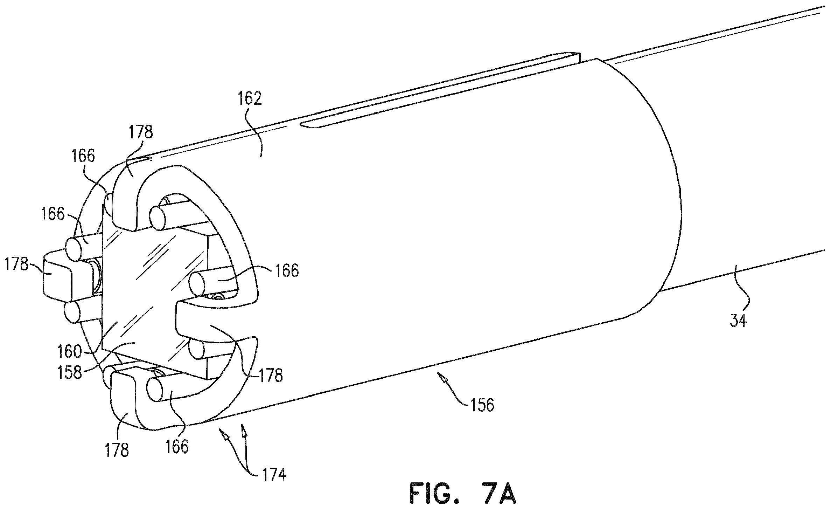

An imaging assembly 156 is fixedly attached to the distal end of the guidewire. Elements of assembly 156 are described with respect to Figs. 7A, 7B, 8, and 9.

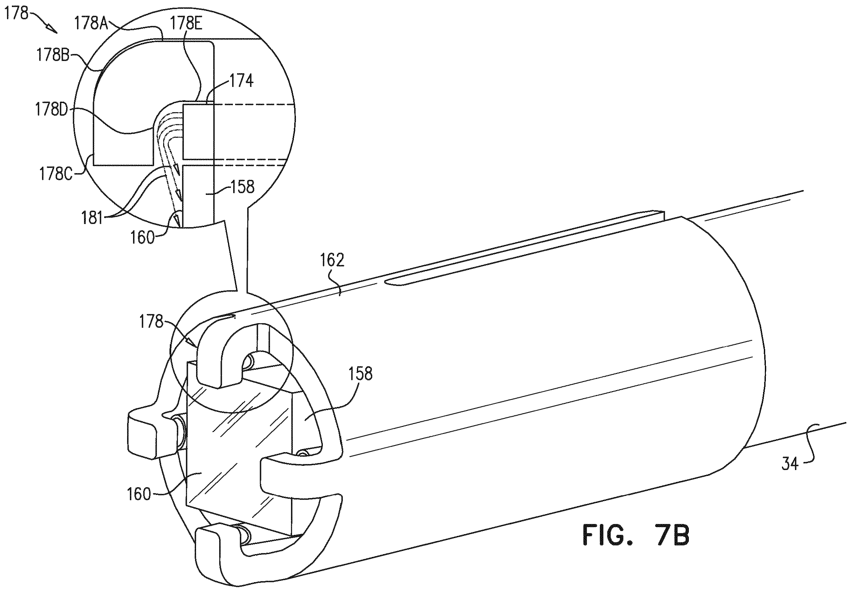

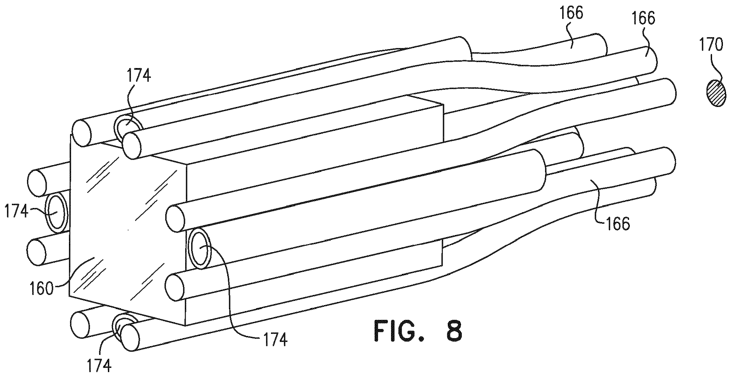

Assembly 156 comprises a camera 158, which is mounted in an assembly enclosure 162, and which has a distal face 160. Enclosure 162 is attached at its proximal side to guidewire 34. In one embodiment camera face 160 is square with a side of 1 mm. In an alternative embodiment face 160 is square with a side of 0.5 mm. Typically the camera has a depth of approximately 2.5 mm. Figs. 7A and 7B show assembly 156 and enclosure 162. Figs. 8 and 9 show assembly 156 with the enclosure hidden. In one embodiment guidewire 34 has a diameter of approximately 2 mm, and enclosure 162 is cylindrical having substantially the same external diameter. However other embodiments have guidewire and enclosure diameters larger or smaller than 2 mm.

In order to illuminate the scene being imaged by the camera, assembly 156 comprises a plurality of optic fibers 166 which surround the camera and which are configured to project light from their distal ends. The optic fibers receive the imaging light at their proximal ends, and the light input to the fibers may be generated by one or more LEDs 170.

In some embodiments fibers 166 are short, having their proximal ends terminating close to the distal end of the guidewire. These embodiments are illustrated in Figs. 8 and 9. In these embodiments one or more LEDs 170 may be located close to the fiber optic proximal ends, the LEDs being powered by conducting lines (not shown in the figures) traversing guidewire 34. Fig. 8 schematically shows one LED 170.

In alternative embodiments fibers 166 are long, terminating in handle 150, and the one or more LEDs 170 are also located in the handle and are configured there to provide imaging light to the optical fiber proximal ends.

In both types of embodiment, controls for the one or more LEDS may be incorporated into handle 150.

Assembly 156 also comprises one or more tubes 174 which terminate distally approximately in line with camera face 160. Tubes 174 are best seen in Figs. 8 and 9. By way of example, assembly 156 is illustrated as having four tubes 174, but embodiments of the present invention may have more or less than four tubes. Each tube traverses guidewire 34, and terminates proximally within handle 150. Within the handle each tube 174 may be supplied with irrigation fluid, which ejects from the tubes at their distal ends. Alternatively, suction may be applied to a given tube 174, so that fluid which the tube distal termination contacts is withdrawn along the tube.

Opposite the distal termination of each tube 174 there is a respective fluid deflector 178 that extends distally from the distal end of enclosure 162. Each deflector 178 is curved, so that fluid exiting in a distal direction from its respective tube 174 strikes the deflector and is deflected approximately orthogonally. The deflected fluid consequently traverses across camera distal face 160, so cleaning the face of the camera.

Fig. 7B illustrates elements of fluid deflector 178. Deflector 178 is formed of three sections: two straight sections 178A, 178C, and a curved section 178B which connects the two straight sections. Straight section 178C has an inner plane surface 178D which is parallel to, and separated from, camera distal face 160. Straight section 178A is physically attached at its proximal side to enclosure 162, at its distal side to curved section 178B, and has an inner plane surface 178E which is orthogonal to camera distal face 160.

When a given tube 174 is configured to eject fluid, the fluid follows a curved path exiting the tube, illustrated by an arrow 181, because the fluid is deflected by the deflector 178, opposite the given tube, to traverse distal face 160.

Alternatively, when a given tube 174 is configured to withdraw fluid, the fluid follows a curved path opposite to that shown by arrow 181, because the withdrawn fluid is also deflected by the deflector 178, and so is removed from distal face 160.

Typically, when guidewire 34 has been inserted into patient 22, the distal end of the guidewire may be surrounded by mucus and/or blood, substantially blinding camera 158. To return the camera to an operative condition, physician 54 may feed fluid into one or more tubes 174, so that the exiting fluid passes over the distal face of the camera, and cleans it. In some embodiments, while some tubes 174 are configured to eject fluid, other tubes 174 may be configured to withdraw fluid.

In a disclosed embodiment, tubes 174 on opposite sides of camera 158 are configured to simultaneously eject and withdraw fluid from the distal face of the camera. For example, in Fig. 7B the upper tube 174 may eject fluid, while the lower tube 174 may simultaneously withdraw fluid. Similarly the tube 174 behind the camera may eject fluid, while the tube 174 in front of the camera simultaneously withdraws fluid. Such simultaneous ejection and withdrawal of fluid from distal face 158 enhances the cleaning of the distal face.

Assembly 156 comprises a magnetic sensor 180, substantially similar to sensor 32, enabling processor 40 (Fig. 1) to track the location and orientation of the distal end of guidewire 34. Alternatively, a magnetic sensor may be provided by winding a coil around enclosure 162.

Conductors 184, from assembly 156, are connected to sensor 180 and to camera 158, and the conductors traverse guidewire 34 terminating at handle 150. The conductors provide power and data signals to the camera, and transfer signals from the camera and from sensor 180 to the handle, Processor 40 is thus able to control the imaging of the camera, and present a scene viewed by the camera on screen 56.

Although the embodiments described herein mainly address improvements in a guidewire for ENT, the methods and systems described herein may also be used in other, non-ENT applications, such as in neuro, gastric and other laparoscopic surgeries.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. Apparatus, comprising:
a guidewire (34) having a proximal end and a distal end which is deflectable;
at least one wire (185) within the guidewire, traversing the guidewire from the proximal end to the distal end, wherein the at least one wire is fixed to the distal end;
one or more irrigation tubes (174) within the guidewire and traversing the guidewire from the proximal end to the distal end; and
an imaging assembly enclosure (162), fixedly attached to the distal end, comprising:
a camera (158), having a distal face (160), fixedly mounted within the enclosure;
respective deflectors (178) for each of the one or more irrigation tubes, fixedly attached to and positioned at a distal end of the enclosure so as to deflect fluid, ejected from the one or more irrigation tubes, to pass over the camera distal face so as to clean the face,
so that applying tension at the proximal end of the guidewire on the at least one wire deflects the distal end of the guidewire, the imaging assembly enclosure attached thereto, and the camera in the enclosure; and
wherein the one or more irrigation tubes comprises at least two irrigation tubes on opposite sides of the camera, **characterised in that** j the at least two irrigation tubes are configured to simultaneously eject and withdraw fluid from the camera distal face.

2. The apparatus according to claim 1, wherein the distal face (160) of the camera (158) is orthogonal to a surface of the enclosure (162), and wherein each of the respective deflectors (178) comprises a first straight section (178A) orthogonal to the distal face of the camera and connected to the distal end of the enclosure, a second straight section (178C) parallel to the distal face of the camera, and a curved section (178B) connecting the first and second straight sections.

3. The apparatus according to claim 2, wherein each of the one or more irrigation tubes (174) has a respective distal end parallel to the first straight section (178A) of the each of the respective deflectors (178).

4. The apparatus according to claim 1, wherein the application of suction to a given tube of the two or more irrigation tubes causes the respective deflector (178) of the given tube to remove fluid from the camera distal face (160), and to direct the fluid into the given tube.

5. The apparatus according to claim 1, and comprising a magnetic sensor, mounted in the imaging assembly enclosure, configured to provide an indication of a location and an orientation of the imaging assembly enclosure in response to a magnetic field traversing the sensor.

6. A method, comprising:
providing a guidewire (34) having a proximal end and a distal end which is deflectable;
positioning at least one wire (185) within the guidewire, to traverse the guidewire from the proximal end to the distal end, wherein the at least one wire is fixed to the distal end;
positioning one or more irrigation tubes (174) within the guidewire to traverse the guidewire from the proximal end to the distal end; and
fixedly attaching an imaging assembly enclosure (162) to the distal end, the assembly comprising:
a camera (158), having a distal face (160), fixedly mounted within the enclosure;
respective deflectors (178) for each of the one or more irrigation tubes, fixedly attached to and positioned at a distal end of the enclosure so as to deflect fluid, ejected from the one or more irrigation tubes, to pass over the camera distal face so as to clean the face,
so that applying tension at the proximal end of the guidewire on the at least one wire deflects the distal end of the guidewire, the imaging assembly enclosure attached thereto, and the camera in the enclosure; and
wherein the one or more irrigation tubes comprises at least two irrigation tubes on opposite sides of the camera, **characterised in that** j the at least two irrigation tubes are configured to simultaneously eject and withdraw fluid from the camera distal face.

7. The method according to claim 6, wherein the distal face of the camera (160) is orthogonal to a surface of the enclosure (162), and wherein each of the respective deflectors (178) comprises a first straight section (178A) orthogonal to the distal face of the camera and connected to the distal end of the enclosure, a second straight section parallel (178C) to the distal face of the camera, and a curved section (178B) connecting the first and second straight sections.

8. The method according to claim 7, wherein each of the one or more irrigation tubes (174) has a respective distal end parallel to the first straight section (178A) of the each of the respective deflectors (178).

9. The method according to claim 6, wherein the one or more irrigation tubes (174) comprise two or more irrigation tubes, the method further comprising applying suction to a given tube of the two or more irrigation tubes so as to cause the respective deflector (178) of the given tube to remove fluid from the camera distal face (160), and to direct the fluid into the given tube.

10. The method according to claim 6, and comprising mounting a magnetic sensor in the imaging assembly enclosure, wherein the sensor is configured to provide an indication of a location and an orientation of the imaging assembly enclosure in response to a magnetic field traversing the sensor.

## Patentansprüche

1. Vorrichtung, umfassend:
einen Führungsdraht (34) mit einem proximalen Ende und einem distalen Ende, das biegsam ist;
mindestens einen Draht (185) innerhalb des Führungsdrahts, der den Führungsdraht von dem proximalen Ende zu dem distalen Ende durchquert, wobei der mindestens eine Draht an dem distalen Ende fixiert ist;
ein oder mehrere Spülrohre (174) innerhalb des Führungsdrahts, und die den Führungsdraht von dem proximalen Ende zu dem distalen Ende durchqueren; und
eine Bildgebungsanordnungseinhausung (162), die fixiert an dem distalen Ende befestigt ist, umfassend:
eine Kamera (158) mit einer distalen Fläche (160), die fixiert innerhalb der Einhausung montiert ist;
jeweilige Deflektoren (178) für jedes von dem einen oder den mehreren Spülrohren, die fixiert an einem distalen Ende der Einhausung befestigt und positioniert sind, um Fluid, das aus dem einen oder den mehreren Spülrohren ausgestoßen wird, abzulenken, so dass es über die distale Fläche der Kamera geführt wird, um so die Fläche zu reinigen,
so dass Anwenden von mechanischer Spannung an dem proximalen Ende des Führungsdrahts auf den mindestens einen Draht das distale Ende des Führungsdrahts, die daran befestigte Bildgebungsanordnungseinhausung und die Kamera in der Einhausung ablenkt; und
wobei das eine oder die mehreren Spülrohre mindestens zwei Spülrohre auf gegenüber liegenden Seiten der Kamera umfasst bzw. umfassen,
**dadurch gekennzeichnet, dass**
die mindestens zwei Spülrohre ausgestaltet sind, um simultan Fluid auszustoßen und von der distalen Fläche der Kamera abzuziehen.

2. Vorrichtung nach Anspruch 1, wobei die distale Fläche (160) der Kamera (158) orthogonal zu einer Oberfläche der Einhausung (162) ist, und wobei jeder der jeweiligen Deflektoren (178) ein erstes gerades Segment (178A), das orthogonal zu der distalen Fläche der Kamera ist und mit dem distalen Ende der Einhausung verbunden ist, ein zweites gerades Segment (178C) parallel zu der distalen Fläche der Kamera und ein gekrümmtes Segment (178B) umfasst, welches das erste und das zweite gerade Segment verbindet.

3. Vorrichtung nach Anspruch 2, wobei jedes von dem einen oder den mehreren Spülrohren (174) ein jeweiliges distales Ende aufweist, das parallel zu dem ersten geraden Segment (178A) von jedem der jeweiligen Deflektoren (178) ist.

4. Vorrichtung nach Anspruch 1, wobei das Anwenden von Saugkraft auf ein gegebenes Rohr der zwei oder mehr Spülrohre bewirkt, dass der jeweilige Deflektor (178) des gegebenen Rohrs Fluid von der distalen Fläche (160) der Kamera entfernt und das Fluid in das gegebene Rohr leitet.

5. Vorrichtung nach Anspruch 1, und umfassend einen Magnetsensor, der in der Bildgebungsanordnungseinhausung montiert ist und konfiguriert ist, um eine Angabe einer Position und einer Orientierung der Bildgebungsanordnungseinhausung in Reaktion auf ein Magnetfeld bereitzustellen, das den Sensor durchquert.

6. Verfahren, umfassend:
Bereitstellen eines Führungsdrahts (34) mit einem proximalen Ende und einem distalen Ende, das biegsam ist;
Positionieren von mindestens einem Draht (185) innerhalb des Führungsdrahts, um den Führungsdraht von dem proximalen Ende zu dem distalen Ende zu durchqueren, wobei der mindestens eine Draht an dem distalen Ende fixiert ist;
Positionieren von einem oder mehreren Spülrohren (174) innerhalb des Führungsdrahts, um den Führungsdraht von dem proximalen Ende zu dem distalen Ende zu durchqueren; und
fixiertes Befestigen einer Bildgebungsanordnungseinhausung (162) an dem distalen Ende, wobei die Anordnung umfasst:
eine Kamera (158) mit einer distalen Fläche (160), die fixiert innerhalb der Einhausung montiert ist;
jeweilige Deflektoren (178) für jedes von dem einen oder den mehreren Spülrohren, die fixiert an einem distalen Ende der Einhausung befestigt und positioniert sind, um so Fluid, das aus dem einen oder den mehreren Spülrohren ausgestoßen wird, abzulenken, so dass es über die distale Fläche der Kamera geführt wird, um so die Fläche zu reinigen,
so dass Anwenden von mechanischer Spannung an dem proximalen Ende des Führungsdrahts auf den mindestens einen Draht das distale Ende des Führungsdrahts, die daran befestigte Bildgebungsanordnungseinhausung und die Kamera in der Einhausung ablenkt; und
wobei das eine oder die mehreren Spülrohre mindestens zwei Spülrohre auf gegenüber liegenden Seiten der Kamera umfassen,
**dadurch gekennzeichnet, dass**
die mindestens zwei Spülrohre ausgestaltet sind, um simultan Fluid auszustoßen und von der distalen Fläche der Kamera abzuziehen.

7. Verfahren nach Anspruch 6, wobei die distale Fläche der Kamera (160) orthogonal zu einer Oberfläche der Einhausung (162) ist, und wobei jeder der jeweiligen Deflektoren (178) ein erstes gerades Segment (178A), das orthogonal zu der distalen Fläche der Kamera ist und mit dem distalen Ende der Einhausung verbunden ist, ein zweites gerades Segment (178C) parallel zu der distalen Fläche der Kamera und ein gekrümmtes Segment (178B) umfasst, welches das erste und das zweite gerade Segment verbindet.

8. Verfahren nach Anspruch 7, wobei jedes von dem einen oder den mehreren Spülrohren (174) ein jeweiliges distales Ende parallel zu dem ersten geraden Segment (178A) von jedem der jeweiligen Deflektoren (178) aufweist.

9. Verfahren nach Anspruch 6, wobei das eine oder die mehreren Spülrohre (174) zwei oder mehr Spülrohre umfasst bzw. umfassen, wobei das Verfahren des Weiteren Anwenden von Saugkraft auf ein gegebenes Rohr der zwei oder mehr Spülrohre umfasst, um zu bewirken, dass der jeweilige Deflektor (178) des gegebenen Rohrs Fluid von der distalen Fläche (160) der Kamera entfernt und das Fluid in das gegebene Rohr leitet.

10. Verfahren nach Anspruch 6, und umfassend Montieren eines Magnetsensors in der Bildgebungsanordnungseinhausung, wobei der Sensor konfiguriert ist, um eine Angabe einer Position und einer Orientierung der Bildgebungsanordnungseinhausung in Reaktion auf ein Magnetfeld bereitzustellen, das den Sensor durchquert.

## Revendications

1. Appareil, comprenant :
un fil-guide (34) ayant une extrémité proximale et une extrémité distale qui peut être déviée ;
au moins un fil (185) à l'intérieur du fil-guide, traversant le fil-guide de l'extrémité proximale à l'extrémité distale, l'au moins un fil étant fixé à l'extrémité distale ;
un ou plusieurs tubes d'irrigation (174) à l'intérieur du fil-guide et traversant le fil-guide de l'extrémité proximale à l'extrémité distale ; et
une enceinte d'ensemble d'imagerie (162), attachée de manière fixe à l'extrémité distale, comprenant :
une caméra (158), ayant une face distale (160), montée de manière fixe dans l'enceinte ;
des déflecteurs respectifs (178) pour chacun du ou des tubes d'irrigation, attachés de manière fixe à et positionnés à une extrémité distale de l'enceinte de manière à dévier le fluide, éjecté du ou des tubes d'irrigation, pour qu'il passe sur la face distale de la caméra afin de nettoyer la face,
de sorte que l'application d'une tension à l'extrémité proximale du fil-guide sur l'au moins un fil dévie l'extrémité distale du fil-guide, l'enceinte de l'ensemble d'imagerie attachée à celui-ci, et la caméra dans l'enceinte ; et
le ou les tubes d'irrigation comprenant au moins deux tubes d'irrigation sur les côtés opposés de la caméra,
**caractérisé en ce que** les au moins deux tubes d'irrigation sont configurés pour éjecter et retirer simultanément du fluide de la face distale de la caméra.

2. Appareil selon la revendication 1, la face distale (160) de la caméra (158) étant orthogonale à une surface de l'enceinte (162), et chacun des déflecteurs respectifs (178) comprenant une première section droite (178A) orthogonale à la face distale de la caméra et reliée à l'extrémité distale de l'enceinte, une seconde section droite (178C) parallèle à la face distale de la caméra, et une section incurvée (178B) reliant les première et seconde sections droites.

3. Appareil selon la revendication 2, chacun du ou des tubes d'irrigation (174) ayant une extrémité distale respective parallèle à la première section droite (178A) de chacun des déflecteurs respectifs (178).

4. Appareil selon la revendication 1, l'application d'une aspiration à un tube donné parmi les deux ou plus de deux tubes d'irrigation amenant le déflecteur respectif (178) du tube donné à retirer du fluide de la face distale de la caméra (160), et à diriger le fluide dans le tube donné.

5. Appareil selon la revendication 1, et comprenant un capteur magnétique, monté dans l'enceinte de l'ensemble d'imagerie, configuré pour fournir une indication d'un emplacement et d'une orientation de l'enceinte de l'ensemble d'imagerie en réponse à un champ magnétique traversant le capteur.

6. Procédé, comprenant :
la fourniture d'un fil-guide (34) ayant une extrémité proximale et une extrémité distale qui peut être déviée ;
le positionnement d'au moins un fil (185) à l'intérieur du fil-guide, pour traverser le fil-guide de l'extrémité proximale à l'extrémité distale, l'au moins un fil étant fixé à l'extrémité distale ;
le positionnement d'un ou plusieurs tubes d'irrigation (174) à l'intérieur du fil-guide pour traverser le fil-guide de l'extrémité proximale à l'extrémité distale ; et
l'attachement de manière fixe d'une enceinte d'ensemble d'imagerie (162) à l'extrémité distale, l'ensemble comprenant :
une caméra (158), ayant une face distale (160), montée de manière fixe dans l'enceinte ;
des déflecteurs respectifs (178) pour chacun du ou des tubes d'irrigation, attachés de manière fixe à et positionnés à une extrémité distale de l'enceinte de manière à dévier le fluide, éjecté du ou des tubes d'irrigation, pour qu'il passe sur la face distale de la caméra afin de nettoyer la face,
de sorte que l'application d'une tension à l'extrémité proximale du fil-guide sur l'au moins un fil dévie l'extrémité distale du fil-guide, l'enceinte de l'ensemble d'imagerie attachée à celui-ci, et la caméra dans l'enceinte ; et
le ou les tubes d'irrigation comprenant au moins deux tubes d'irrigation sur des côtés opposés de la caméra,
**caractérisé en ce que** les au moins deux tubes d'irrigation sont configurés pour éjecter et retirer simultanément un fluide de la face distale de la caméra.

7. Procédé selon la revendication 6, la face distale de la caméra (160) étant orthogonale à une surface de l'enceinte (162), et chacun des déflecteurs respectifs (178) comprenant une première section droite (178A) orthogonale à la face distale de la caméra et reliée à l'extrémité distale de l'enceinte, une seconde section droite parallèle (178C) à la face distale de la caméra, et une section incurvée (178B) reliant les première et seconde sections droites.

8. Procédé selon la revendication 7, chacun du ou des tubes d'irrigation (174) ayant une extrémité distale respective parallèle à la première section droite (178A) de chacun des déflecteurs respectifs (178).

9. Procédé selon la revendication 6, le ou les tubes d'irrigation (174) comprenant deux ou plus de deux tubes d'irrigation, le procédé comprenant en outre l'application d'une aspiration à un tube donné des deux ou plus de deux tubes d'irrigation de manière à amener le déflecteur respectif (178) du tube donné à retirer du fluide de la face distale de la caméra (160), et à diriger le fluide dans le tube donné.

10. Procédé selon la revendication 6, et comprenant le montage d'un capteur magnétique dans l'enceinte de l'ensemble d'imagerie, le capteur étant configuré pour fournir une indication d'un emplacement et d'une orientation de l'enceinte de l'ensemble d'imagerie en réponse à un champ magnétique traversant le capteur.
